Europäisches Patentamt

**European Patent Office**

**Office européen des brevets**

(11) Numéro de publication: **0 097 109**
**A1**

# DEMANDE DE BREVET EUROPEEN

(21) Numéro de dépôt: **83450015.9**

(22) Date de dépôt: **08.06.83**

(51) Int. Cl.³: **A 61 L 2/06**, A 23 L 3/02

(30) Priorité: **10.06.82 EP 82450009**

(43) Date de publication de la demande: **28.12.83**
**Bulletin 83/52**

(84) Etats contractants désignés: **AT BE CH DE FR GB IT LI NL SE**

(71) Demandeur: **Baguet, Paul, 10 avenue des Mésanges, F-33320 Le Taillan (FR)**

(84) Etats contractants désignés: **AT BE CH DE FR GB IT LI NL SE**

(71) Demandeur: **S.A.R.L. COMMODORE INTERNATIONAL, Complexe Artisano Industriel Secteur U, F-33290 Blanquefort (FR)**

(84) Etats contractants désignés: **DE FR**

(72) Inventeur: **Baguet, Paul, 10 avenue des Mésanges, F-33320 Le Taillan (FR)**

(74) Mandataire: **Thébault, Jean-Louis, Cabinet Jean-Louis Thébault 50, Cours de Verdun, F-33000 Bordeaux (FR)**

(54) **Dispositif de stérilisation de récipients contenant de l'eau.**

(57) L'invention concerne un dispositif de stérilisation de récipients contenant en partie de l'eau et/ou des solides mouillés, en particulier des biberons, caractérisé en ce qu'il comporte une enceinte isothermique (1) fermée et mise à l'atmosphère, des moyens de chauffage (5) disposés à l'intérieur de l'enceinte, des moyens (6) de régulation de la température du fluide caloporteur, en l'occurrence essentiellement de l'air, susceptibles de maintenir constamment ladite température dans une plage déterminée de températures, des moyens pour stocker les récipients (7) à l'intérieur de l'enceinte et des moyens susceptibles de permettre l'introduction et l'extraction un à un des récipients (7) de manière à faire correspondre une introduction d'un nouveau récipient à une extraction de celui de récipients ayant séjourné le plus longtemps dans l'enceinte.

Application notamment aux biberons.

- 1 -

## DISPOSITIF DE STERILISATION DE RECIPIENTS CONTENANT DE L'EAU

La présente invention a trait à la stérilisation de récipients contenant en partie de l'eau ou éventuellement des solides mouillés et plus particulièrement, bien que non exclusivement, à la stérilisation de biberon préalablement rempli de l'eau nécessaire à la confection du biberon.

D'une façon générale, la stérilisation mise en oeuvre dans l'invention est une stérilisation de produits contenant de l'eau non sensibles à la température de 100°C et pouvant supporter des valeurs stérilisatrices efficaces calculées suivant la formule connue :

$$Fo = 10^{(\frac{T1 - 121,111}{Z})} \times t$$

dans laquelle :

T1 = Température de stérilisation

Z = 10°C

t = nombre de minutes d'action de la chaleur.

Il existe actuellement deux procédés de stérilisation de produits contenant de l'eau :

1) Une stérilisation "douce", dite aussi "optimale", dans laquelle on recherche d'abord le degré de contamination que peut avoir le produit après fabrication et avant stérilisation (en recherchant évidemment les meilleures conditions de fabrication donnant le minimum de contamination) et à partir de laquelle on adapte la valeur de Fo la plus petite possible pour dégrader le moins possible le produit.

2) Une stérilisation "massive" qui détruit avec certitude les germes les plus résistants grâce à des valeurs stérilisatrices importantes supérieures à 8.

L'invention se rapporte à la seconde méthode et vise à obtenir à la pression atmosphérique une véritable stérilisation, la température restant au voisinage de 100°C, en évitant le recours à des appareils à pression onéreux et gros consommateurs de vapeur et donc d'énergie.

L'invention sera décrite dans son application à la stérilisation de biberons bien qu'elle puisse être appliquée à la stérilisation d'autres produits comme on le verra plus loin.

Dans le cadre de cette application aux biberons, il existe actuellement trois méthodes de stérilisation des biberons :

1) Stérilisation des biberons vides ou pleins avec les accessoires dans un autoclave en bon état de marche. Ce procédé est sûr car la valeur stérilisatrice est généralement importante. Toutefois, compte tenu du coût d'un autoclave et de son utilisation, en fait seul un petit nombre de collectivités par rapport à la totalité utilise ce procédé, et aucun particulier.

2) "Stérilisation" des biberons vides avec les accessoires dans un faitout baptisé pour la circonstance "stérilisateur". Ce procédé malgré une heure de maintien de l'ébullition ne stérilise pas car on obtient une valeur stérilisatrice Fo de 0,5 environ en une heure, et les tests bactériologiques le prouvent largement. Les tests bactériologiques montrent une non stérilisation après 3 heures de maintien de l'ébullition, alors que dans la pratique le temps d'ébullition ne dépasse pas une heure.

3) "Stérilisation" des biberons vides avec les accessoires dans un bain de solution antiseptique généralement à base de chlore, pendant 90 minutes selon les notices. Ce procédé est complètement inefficace dès que les germes sont un peu résistants. On ne compte plus les épidémies dans les collectivités qui l'emploient à cause de sa commodité, et de l'absence d'un moyen réellement efficace et économique.

Le but de l'invention est de proposer un dispositif capable d'assurer une réelle et efficace stérilisation des bi-

berons dans des conditions particulièrement économiques.

A cet effet, l'invention a pour objet un dispositif de stérilisation de récipients contenant en partie de l'eau et/ou des solides mouillés, en particulier des biberons, caractérisé en ce qu'il comporte une enceinte isothermique fermée et mise à l'atmosphère, des moyens de chauffage disposés à l'intérieur de l'enceinte, des moyens de régulation de la température du fluide caloporteur, en l'occurrence essentiellement de l'air, susceptibles de maintenir constamment ladite température dans une plage déterminée de températures, des moyens pour stocker les récipients à l'intérieur de l'enceinte et des moyens susceptibles de permettre l'introduction et l'extraction un à un des récipients de manière à faire correspondre une introduction d'un nouveau récipient à une extraction de celui des récipients ayant séjourné le plus longtemps dans l'enceinte.

Dans un tel dispositif, en prévoyant une capacité de stockage de six ou huit biberons par exemple et en faisant fonctionner en permanence jour et nuit les moyens de chauffage, on peut assurer aux biberons une stérilisation absolument totale puisque le temps de séjour de chaque biberon à l'intérieur de l'enceinte thermostatée, entre le moment où il est introduit et celui où il en est extrait, est de plusieurs heures, les biberons étant prélevés dans le dispositif un à un à chaque tétée suivant un cycle tel que le dernier biberon introduit ne sera extrait qu'une fois que tous les autres biberons qui l'ont précédé dans le dispositif auront été un à un retirés.

On a ainsi l'assurance que chaque biberon extrait a passé plusieurs heures à l'intérieur du dispositif et a donc subi une stérilisation véritable et complète à la température requise pendant un temps largement supérieur à ce qui est normalement nécessaire.

D'autres caractéristiques et avantages ressortiront de la description qui va suivre de modes de réalisation du dispositif selon l'invention, description donnée à titre d'exemple uniquement et en regard des dessins annexés sur lesquels:

- Fig. 1 représente une vue de dessus schématique d'un mode de réalisation d'un dispositif conforme à l'invention appliqué à la stérilisation des biberons,

- Fig. 2 représente une vue en coupe verticale axiale partielle suivant la ligne II-II du dispositif de la Fig. 1,

- Fig. 3 et 4 illustrent schématiquement deux modes de réalisation à enceinte isothermique rectiligne,

- Fig. 5 représente une vue en coupe verticale axiale d'une variante de réalisation du dispositif des Fig. 1 et 2,

- Fig. 6 est une vue en élévation de la couronne mobile de sélection du dispositif de la Fig. 5, et

- Fig. 7 représente une vue de dessus partielle en coupe suivant la ligne VII-VII du dispositif de la Fig. 6.

Le dispositif représenté sur les dessins est constitué d'une enceinte isothermique 1 de forme générale cylindrique réalisée en un matériau de préférence ininflammable et excellent isolant thermique telle que de la laine de verre ou de roche par exemple.

L'enceinte 1 est complètement fermée excepté un passage circulaire 2 dans l'axe vertical de l'enceinte et un orifice 3 d'introduction/extraction des biberons à l'intérieur de l'enceinte. L'enceinte n'est pas toutefois isolée de l'extérieur et contient en permanence de l'air à la pression atmosphérique.

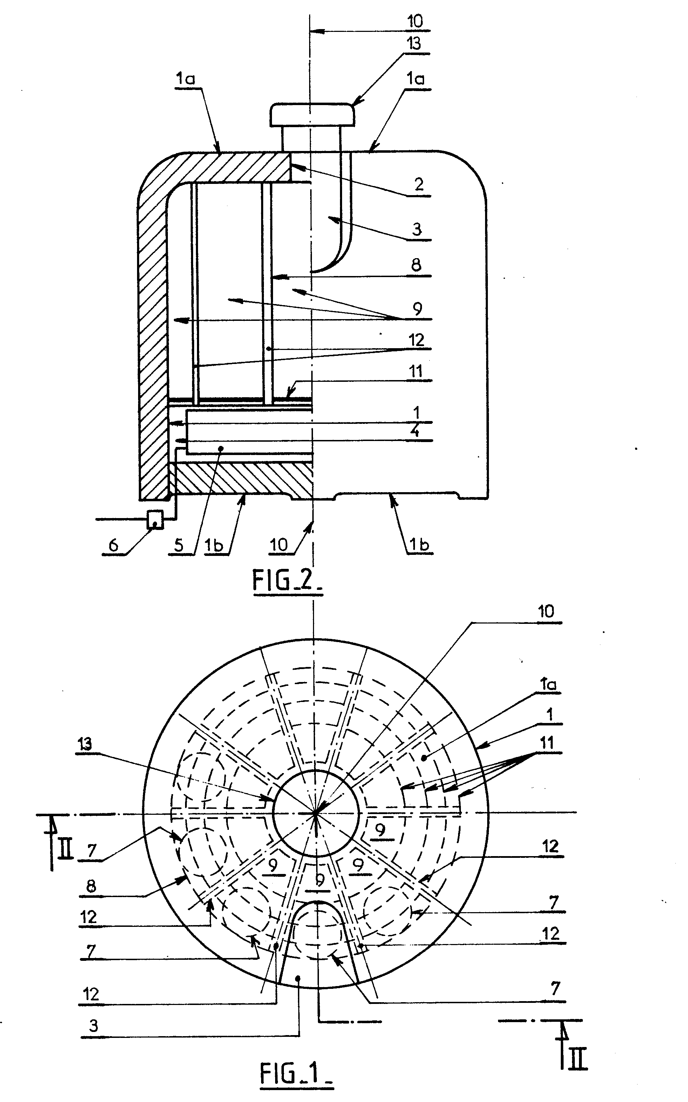

L'orifice 3 s'étend sur la partie extérieure d'un secteur angulaire (Fig. 1) correspondant à un compartiment à biberon pour ce qui concerne la paroi circulaire sommitale 1a de l'enceinte 1 et la partie supérieure correspondante de la paroi latérale cylindrique.

L'intérieur de l'enceinte 1 est divisé en deux parties, une partie inférieure 4 dans laquelle est monté un moyen de chauffage symbolisé en 5 disposé au voisinage de la paroi circulaire de fond 1b de l'enceinte 1. Ce moyen de chauffage 5 est par exemple un dispositif à résistance chauffante électrique alimenté en courant électrique par l'intermédiaire d'un dispositif de régulation de température 6 (thermostat) réglable, branché en permanence sur une source de courant électrique appropriée.

Le dimensionnement des parois de l'enceinte 1 et les caractéristiques du dispositif à résistance chauffante 5 et du régulateur 6 sont telles que le dispositif étant branché en permanence, il est possible de maintenir en permanence 24 heures sur 24 à l'intérieur de l'enceinte thermostatée 1 une température se situant à l'intérieur d'une plage relativement réduite et précise (par exemple entre 100 et 110°C correspondant à une température à l'intérieur des biberons légèrement inférieure à 100°C) avec des coûts de revient particulièrement économiques puisque pratiquement insignifiants du fait de l'excellente isolation thermique et de l'utilisation de l'air comme fluide caloporteur à l'intérieur de l'enceinte.

L'air est, en effet, le seul fluide caloporteur utilisé à l'intérieur de l'enceinte et en contact avec les parois externes des récipients à stériliser même si ces derniers sont chauffés par des parties solides ou en contact avec des parties solides, du fait de la subsistance d'une couche-limite entre les récipients et lesdites parties. L'air a une faible chaleur spécifique et sa mise en oeuvre comme fluide caloporteur est particulièrement avantageuse au niveau, d'une part, de la consommation d'énergie et, d'autre part, de la facilité et du faible coût de la régulation en température. La consommation est en fait limitée à l'énergie nécessaire pour porter le contenu des biberons de 20° à 99°C environ et pour compenser la déperdition extérieure des biberons de l'ordre de quelques watts par m² et par heure.

Au dessus de la partie 4 s'étend une chambre dans laquelle sont stockés les biberons 7 à stériliser et conserver en température. Les biberons 7 sont stockés dans une sorte de panier-magasin circulaire 8 à compartiments rayonnants 9 dans chacun desquels un biberon 7 peut être logé. Le panier-magasin 8 constitue en quelque sorte un barillet mobile autour de l'axe vertical 10 du dispositif.

A cet effet, le panier-magasin 8 est formé par exemple d'une grille circulaire de fond 11 sur laquelle reposent les biberons 7 placés verticalement, un dans chaque compartiment, la délimitation entre compartiments contigus étant faite par une paroi verticale 12 de séparation.

Le panier-magasin 8 est solidaire à sa partie supérieure d'une poignée 13 de commande de rotation, extérieure à l'enceinte 1 et reliée au panier-magasin 8.

La rigidité de l'enceinte 1 est assurée par des moyens appropriés ainsi que la suspension du panier-magasin 8 de manière que ce dernier puisse librement être tourné à la main afin de présenter devant l'orifice 3 le compartiment 9 désiré.

L'orifice 3 est obturé par un couvercle ou dispositif de fermeture amovible (non représenté) de configuration et structure appropriées.

Ce dispositif d'obturation épousant le plus étroitement possible ledit orifice 3 est seulement retiré juste pour l'introduction d'un biberon dans un compartiment 9 libre ou pour l'enlèvement d'un biberon au moment de la tétée.

Dans le mode de réalisation de la Fig. 1 il est prévu dix compartiments 9 mais ce nombre pourrait bien entendu être diminué ou au contraire augmenté.

La stérilisation des biberons (avec leur eau et leur tétine à l'intérieur) se fait à une température relativement modérée (inférieure à 100°C) par rapport aux températures habituelles de stérilisation mais, étant donné le temps de séjour de plusieurs heures de chaque biberon dans le dispositif, on obtient néanmoins une stérilisation totale.

L'introduction d'un biberon se fait par l'orifice 3 en présentant un compartiment 9 vide par rotation à la main du panier-magasin 8. Ce dernier est manoeuvré toujours dans le même sens de rotation suivant la flèche de la Fig. 1 en sorte que le biberon qui vient d'être introduit n'en sera extrait qu'une fois que tous ceux qui occupent les compartiments précédents auront été retirés. Comme un biberon est retiré à chaque tétée, le temps de séjour dans le dispositif de chaque biberon est donc de plusieurs heures.

Par mesure de sécurité, avant chaque prise de biberon, le panier-magasin 8 doit être rechargé d'un nouveau biberon à stériliser. Il est intéressant de prévoir une isolation de la zone en regard de l'orifice 3 c'est-à-dire corresp..dant au compartiment 9 se trouvant en regard dudit orifice. Dans cette zone moins chauffée, le biberon peut se refroidir pendant les trois heures par exemple qui lui restent à séjourner dans l'appareil avant son extraction pour la tétée suivante. Cela permet le mélange de l'eau chaude avec du lait en poudre froid et d'avoir un mélange aux environs de 37°C au moment de l'utilisation.

Il est à noter que le dispositif de l'invention pourrait être utilisé pour la stérilisation et/ou le maintien en température de tous récipients contenant de l'eau.

C'est ainsi que le dispositif de l'invention pourrait être intégré dans une chaîne de fabrication, par exemple de flacons de sérum physiologique dans laquelle les flavons une fois remplis seraient introduits, au fur et à mesure de leur fabrication, un à un dans un dispositif conforme à l'invention sans rupture de cadence. On éviterait ainsi le stockage intermédiaire et donc la recontamination entre la fabrication et la stérilisation ce qui évite la formation des pyrogènes. Le conditionnement continu après la stérilisation en serait également facilité.

Dans le cadre de l'application à d'autres types de récipients que des biberons (de même d'ailleurs que dans cette dernière application) il est à souligner que la température de stérilisation à l'intérieur des récipients pourrait dépasser 100°C et atteindre 102 à 104°C par exemple pour les biberons et même davantage dans d'autres applications.

Les formes, dimensions, structures et configuration de l'enceinte isothermique 1 et du ou des orifices d'accès et de ses moyens d'obturation peuvent varier dans de larges mesures.

La source de chauffage 5 peut être réalisée par tous moyens appropriés, qu'ils soient électriques ou non.

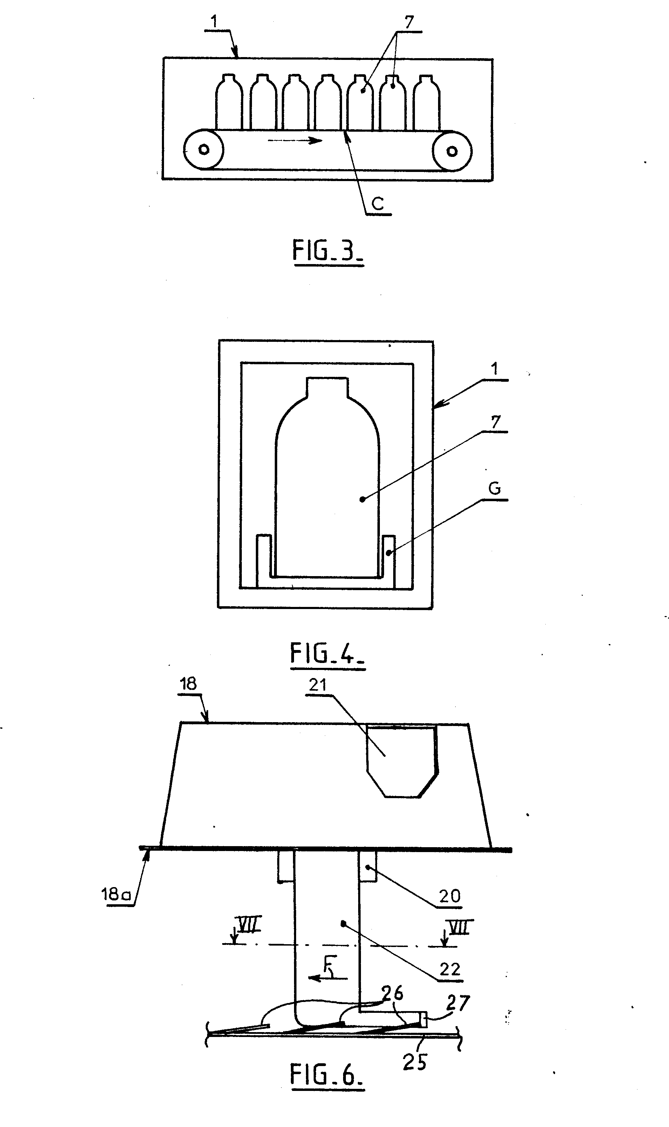

Enfin, le panier-magasin 8 pourrait avoir une autre structure pourvu qu'il permette de présenter devant un ou plusieurs orifices d'introduction/extraction ménagés dans l'enceinte thermostatée, un par un les objets stérilisés ou les emplacements de réception des objets à stériliser, quelle que soit la trajectoire de déplacement. C'est ainsi que l'on peut prévoir une enceinte 1 droite (Fig. 3 et 4) dans laquelle les biberons 7 se suivraient les uns les autres (ou se pousseraient). Les moyens de support des biberons 7 seraient par exemple (Fig. 3 un moyen de convoyage sans fin C ou bien (Fig. 4) un simple chemin de guidage G fixe dans le cas où les biberons 7 se pousseraient les uns les autres à la queue leu leu.

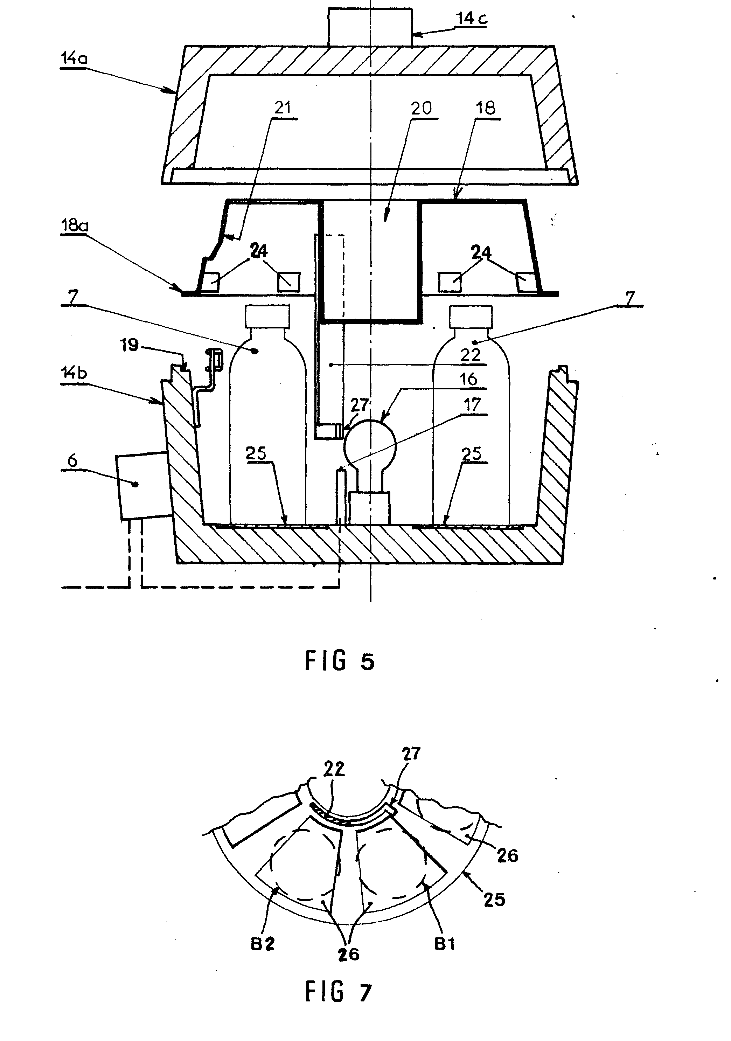

Les Fig. 5 à 7 illustrent une variante de réalisation du dispositif selon l'invention.

Selon cette variante l'enceinte isothermique comprend deux demi-coques indépendantes 14a et 14b posées l'une sur l'autre. La demi-coque inférieure 14b reçoit les biberons cependant que la demi-coque supérieure 14a fait office de couvercle et est munie d'une poignée de préhension 14c.

Les biberons 7 sont disposés en couronne et, dans l'espace central au milieu des biberons est disposé un moyen de chauffage constitué par exemple par une simple ampoule électrique 16 alimentée en courant à partir d'une source appropriée par l'intermédiaire du régulateur 6 lui-même relié à une sonde thermostatique 17 disposée au voisinage de l'ampoule 16.

Dans ce mode de réalisation ce n'est plus l'ensemble des biberons que l'on fait défiler un à un devant une ouverture fixe de prélèvement ou d'introduction, mais le contraire. Les biberons sont immobiles à l'intérieur de l'enceinte 14a-14b et le système de prélèvement et d'introduction des biberons est mobile et constitué par une couronne rotative 18 en forme générale de moule à savarin coiffant l'alignement circulaire des têtes de biberons lorsque le rebord inférieur circulaire externe 18a de la couronne 18 repose sur un épaulement circulaire 19 de réception ménagé à cet effet à la partie supérieure de la demi-coque 14b.

La couronne 18 comporte un puits cylindrique central 20 à l'aplomb de l'ampoule 16 et dont le rôle sera précisé plus loin.

La couronne 18 comporte sur sa périphérie externe une découpe 21 de dimensions suffisantes pour permettre le passage d'un biberon.

Le puits central 20 est prolongé par une palette verticale incurvée 22 disposée du côté de la découpe 21 mais pas en face de celle-ci. Elle est en effet décalée angulairement d'un intervalle correspondant à l'intervalle entre deux biberons 7 consécutifs disposés dans la demi-coque 14b.

L'extrémité inférieure de la palette 22 se trouve à faible distance du fond de la demi-coque 14b lorsque la couronne 18 repose sur l'épaulement circulaire 19. La largeur de la palette 22 correspond sensiblement au diamètre des biberons 7.

La couronne 18 peut glisser librement sur l'épaulement 19 en la faisant tourner à la main de manière à amener la découpe 21 en face de successivement tous les biberons 7.

Un système anti-retour permet la rotation de la couronne 18 sur la demi-coque 14b toujours dans le même sens (flèche F Fig. 6). Ce système est par exemple constitué par un dispositif à cliquet escamotable 23 solidaire de la demi-coque 14b et coopérant avec un jeu de butées 24 régulièrement réparties sur la face interne de la couronne 18. Le nombre des butées 24 est égal à celui des biberons pouvant être placés dans le dispositif. Dans un sens de rotation de la couronne 18 les butées 24 échappent au cliquet anti-retour 23 alors que dans l'autre sens, les butées sont arrêtées par ledit cliquet. Les organes 23 et 24 sont disposés de manière que l'effet de cliquet anti-retour se manifeste dès qu'un biberon 7 (ou emplacement vide de l'enceinte isothermique) se présente à l'aplomb de la découpe 21.

Le dispositif comporte, en outre, un second dispositif détrompeur destiné à permettre la rotation de la couronne 18 seulement si l'emplacement en regard de la découpe 21 est occupé par un biberon. A cet effet, les biberons 7 reposent sur une plaque mince circulaire en forme de rondelle 25 (Fig. 7) dans laquelle sont découpées des lamelles élastiques 26 qui font normalement saillies (Fig. 6) au dessus du plan de la plaque 25 et qui, lorsqu'un biberon repose dessus, se remet dans le plan de la plaque. La couronne 18 qui ne peut se déplacer que dans le sens de la flèche F de la Fig. 6 est bloquée si aucun biberon ne repose sur la lamelle 26 se trouvant à l'aplomb de la découpe 21 (emplacement B1, Fig. 7).

La palette 22 vient, en effet, buter par l'intermédiaire d'un ergot ou saillie 27 fixé latéralement à la palette et à son extrémité inférieure. Cet ergot 27 est déporté latéralement à la palette 22 de façon à venir buter contre le rebord extrême relevé de ladite lamelle 26 à l'aplomb de la découpe 21. Par contre, dès qu'un biberon 7 est posé sur l'emplacement B1, la lamelle 26 associée s'escamote et libère l'ergot 27.

La couronne 18 peut alors être tournée d'un cran pour présenter à l'aplomb de la découpe 21 l'emplacement suivant B2. Il existe bien entendu autant de lamelles 26 que d'emplacements à biberons.

Au moment de la tétée, on enlève le couvercle 14a et on commence par mettre en place dans l'emplacement (qui est supposé vide) en regard de la découpe 21 un biberon neuf à stériliser. Ceci permet, comme on l'a expliqué plus haut, de faire tourner la couronne 18 d'un cran pour accéder à un biberon stérilisé lequel est précisément celui qui a séjourné le plus longtemps dans le dispositif. Ce biberon se trouve à une température moindre que celle des autres biberons du fait du rôle d'écran thermique de la palette 22 qui est demeurée interposée entre lui et la source de chaleur rayonnante 16 jusqu'au moment de la rotation de la couronne 18.

Il est très important de noter que les biberons 7 dans le mode de réalisation de la Figure 5 comme dans celui des figures 1 et 2, doivent contenir au moins 80 à 90% de volume d'eau et être obturés de manière étanche.

De même que dans le cas des figures 1 et 2 la température de stérilisation à l'intérieur des biberons peut dépasser 100°C et atteindre 102 à 104°C, voire même davantage en fonction de la résistance mécanique du biberon ou récipient.

Le puits 20 peut servir soit à réchauffer ou maintenir à température un liquide, une substance, un objet quelconque, soit à stériliser intérieurement par exemple l'eau du jus de fruit destiné au bébé.

REVENDICATIONS
:=:=:=:=:=:=:=:=:=:=:=:=:=:

1. Dispositif de stérilisation de récipients contenant en partie de l'eau et/ou des solides mouillés, en particulier des biberons, caractérisé en ce qu'il comporte une enceinte isothermique (1) fermée et mise à l'atmosphère, des moyens de chauffage (5) disposés à l'intérieur de l'enceinte, des moyens (6) de régulation de la température du fluide caloporteur, en l'occurrence essentiellement de l'air, susceptibles de maintenir constamment ladite température dans une plage déterminée de températures, des moyens pour stocker les récipients (7) à l'intérieur de l'enceinte et des moyens susceptibles de permettre l'introduction et l'extraction un à un des récipients (7) de manière à faire correspondre une introduction d'un nouveau récipient à une extraction de celui des récipients ayant séjourné le plus longtemps dans l'enceinte.

2. Dispositif suivant la revendication 1, caractérisé en ce que la paroi de l'enceinte (1) comporte un orifice de passage (3) muni d'un dispositif de fermeture amovible et en ce que lesdits moyens de stockage des récipients (7) et d'in-troduction/extraction sont constitués par un support (8) de récipients susceptible de permettre le cheminement pas à pas de ces derniers à l'intérieur de l'enceinte de manière que chaque introduction d'un nouveau récipient permette l'extraction de celui des récipients ayant séjourné le plus longtemps dans l'enceinte.

3. Dispositif suivant la revendication 2, caractérisé en ce que ledit support de récipient (7) est constitué par un panier-magasin (8) cylindrique, d'axe vertical et monté rotatif à l'intérieur de l'enceinte (1), la commande de rotation s'effectuant depuis l'extérieur par tous moyens appropriés.

4. Dispositif suivant la revendication 3, caractérisé en ce que le panier-magasin (8) est constitué d'une grille de fond circulaire (11) et de cloisons rayonnantes (12) délimitant un certain nombre d'emplacements ou logements (9) de réception des récipients (7).

5. Dispositif suivant la revendication 4, caractérisé en ce que les moyens de commande de rotation du panier-magasin (8) sont constitués par une poignée, bouton, ou volant (13) extérieur à l'enceinte (1), disposé sur la face supérieure de celle-ci et solidaire en rotation dudit panier-magasin, lesdits moyens étant agencés de manière à ne permettre la rotation que dans un seul sens.

6. Dispositif suivant la revendication 2, caractérisé en ce que ladite enceinte (1) est rectiligne et le support de récipients est un moyen de convoyage sans fin (C) traversant d'un bout à l'autre ladite enceinte, laquelle comporte un orifice d'entrée et un orifice de sortie aux deux extrémités de l'enceinte.

7. Dispositif suivant la revendication 2, caractérisé en ce que ladite enceinte (1) est rectiligne, comporte à ses deux extrémités un orifice d'entrée et un orifice de sortie et est munie intérieurement d'un chemin de guidage fixe (G) canalisant les uns derrière les autres les récipients.

8. Dispositif suivant la revendication 1, caractérisé en ce que l'enceinte comporte deux parties emboîtables dont l'une (14a) forme couvercle et l'autre (14b) forme corps principal, en ce que lesdits moyens de stockage des récipients (7) sont constitués par ledit corps principal (14b), les récipients reposant sur le fond de ce dernier en étant alignés en cercle et en ce que lesdits moyens d'introduction/extraction sont constitués par une couronne mobile (18) disposée à l'intérieur de l'enceinte, au dessus des récipients (7) et munie d'un orifice (21) de passage susceptible de venir à l'aplomb successivement de chaque récipient lors de la rotation de la couronne (18), des moyens (23,24) étant prévus pour permettre ladite rotation dans un sens seulement.

9. Dispositif suivant l'une des revendications 1 à 7, caractérisé en ce que lesdits moyens de chauffage (5) sont constitués par une ou plusieurs résistances électriques branchées en permanence sur une source de courant appropriée par l'intermédiaire desdits moyens de régulation (6).

10. Dispositif suivant les revendications 1 et 8 caractérisé en ce que lesdits moyens de chauffage sont constitués par une ampoule électrique (16) disposée dans l'espace central délimité par les récipients (7) et branchée en permanence sur une source de courant appropriée par l'intermédiaire desdits moyens de régulation (6).

11. Dispositif suivant les revendications 8 et 10 caractérisé en ce que la couronne mobile (18) d'introduction/ extraction est munie d'un écran thermique (22) interposé entre la source centrale de chaleur (16) et le récipient (7) situé immédiatement à l'aval dudit orifice de passage (21) suivant le sens de déplacement de ce dernier.

12. Dispositif suivant la revendication 8, caractérisé en ce que la couronne (18) d'introduction/extraction comporte un puits central (20) pouvant servir de logement de réception d'un objet ou substance à réchauffer, maintenir au chaud ou stériliser.

13. Dispositif suivant l'une des revendications 8 à 12 caractérisé en ce qu'il comporte des moyens (26,27) pour n'autoriser la rotation de la couronne (18) d'introduction/extraction que lorsque l'emplacement situé à l'aplomb de l'orifice de passage (21) est occupé par un récipient.

0097109

FIG. 2.

FIG. 1.

FIG.3.

FIG.4.

FIG.6.

FIG 5

FIG 7

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | FR-A-2 150 858 (SCHIEDAMSCHE WERKTUIGEN EN MACHINEFABRIEK) * Revendications; page 6, lignes 38-40 * | 1 | A 61 L 2/06<br>A 23 L 3/02 |
| A | FR-A-1 266 869 (HOECHST) * Résumé * | 1 | |
| A | FR-A- 541 897 (NIELSEN) * En entier * | 1 | |

-----

**DOMAINES TECHNIQUES RECHERCHES (Int. Cl. 3)**

A 61 L
A 23 L

Le présent rapport de recherche a été établi pour toutes les revendications

| Lieu de la recherche<br>LA HAYE | Date d'achèvement de la recherche<br>05-09-1983 | Examinateur<br>MALHERBE Y.J.M. |
|---|---|---|